# DEMANDE DE BREVET EUROPEEN

(11) **EP 1 124 127 A1**
(43) Date de publication de la demande: **16.08.2001**
(21) Numéro de dépôt: 01400290.1
(22) Date de dépôt: 06.02.2001
(51) Int. Cl.: G01N 21/35, G01N 33/14

(54) **Dispositif de détermination de l'origine d'un produit naturel ou élaboré**

(30) Priorité: 11.02.2000 FR 0001749
(71) Demandeur: THOMSON-CSF, 75008 Paris (FR)
(72) Inventeur: Larive, Marc, Thomson-SCF Propriete Intellectuelle, 94117 Arcueil Cedex (FR); Nory, Pierre, Thomson-SCF Propriete Intellectuelle, 94117 Arcueil Cedex (FR)
(74) Mandataire: Albert, Claude

(57) **Abrégé**

L'invention concerne un dispositif de détermination de l'origine d'un produit naturel ou élaboré par estimation d'au moins un rapport isotopique dudit produit. Selon une variante, il comprend :
- un spectromètre infrarouge (SPEC) permettant de déterminer les caractéristiques spectrales d'au moins une première liaison chimique du produit ou d'un de ses sous-produits, et celles d'au moins une liaison associée à la première liaison, c'est-à-dire comprenant un isotope d'au moins un des atomes formant ladite première liaison,
- des premiers moyens de comparaison (CMP₁) desdites caractéristiques spectrales à une première base de données (BdD₁) comprenant les caractéristiques spectrales de ladite première liaison chimique et de la liaison associée, afin d'établir au moins un rapport isotopique égal au rapport des quantités de sous-produit et de sous-produit associé composant ledit produit et comprenant respectivement ladite première liaison et ladite liaison associée,
- des seconds moyens de comparaison (CMP₂) dudit rapport isotopique à une seconde base de données (BdD₂) comprenant des valeurs dudit rapport isotopique pour des produits ou sous-produits d'origine connue, afin de déterminer l'origine dudit produit.

## Description

L'invention concerne un dispositif de détermination de l'origine d'un produit naturel ou élaboré par estimation d'au moins un rapport isotopique dudit produit. La présente invention permet par exemple l'authentification de produits agro-alimentaires tels que le vin, les alcools, les huiles, etc., tout comme la détermination de l'origine géographique de minerais, de produits dérivés du pétrole, etc.

L'étude de l'authenticité des produits et de leur traçabilité, c'est à dire la connaissance de leur origine géographique, l'analyse de leur constitution lorsqu'ils proviennent de mélanges, éventuellement la détermination de la date de leur extraction lorsqu'il s'agit de produits naturels, est une préoccupation grandissante des consommateurs, des organismes publiques de contrôle, par conséquent, des acteurs de la chaîne de distribution et d'élaboration des produits.

Actuellement, une méthode développée par le professeur G.J.Martin (voir par exemple le brevet US 4,550,082) est utilisée pour l'authentification des vins et la détection de la chaptalisation, c'est à dire l'enrichissement du vin par du sucre. Cette méthode est basée sur une technique d'analyse RMN (abréviation de 'Résonance Magnétique Nucléaire') de l'alcool éthylique contenu dans le vin. La RMN est fondée sur l'analyse des signaux émis par des noyaux de molécules orientées dans un champ magnétique intense et excitées par un champ électromagnétique. Ces signaux permettent d'analyser les structures moléculaires de diverses espèces chimiques, et notamment de déterminer le taux de deutérium (isotope de l'hydrogène), ainsi que le taux de certains autres isotopes tels que ceux du carbone (carbone 13) et de l'oxygène (oxygène 18). Or une analyse expérimentale a permis de constater que l'enrichissement du vin par du sucre de betterave par exemple se repère notamment par la modification du rapport isotopique deutérium/hydrogène dans les différentes liaisons de l'alcool éthylique. Une analyse RMN accompagnée d'un traitement logiciel opérant une comparaison avec une banque de données contenant les taux isotopiques de différents vins d'origine connue permet donc de reconstituer la composition du vin et l'origine de ses constituants.

L'analyse RMN présente cependant un certain nombre d'inconvénients. En particulier, cette technique met en oeuvre des moyens compliqués, très encombrants et fort coûteux qui obligent les utilisateurs potentiels à confier l'analyse à un laboratoire spécialisé possédant l'instrument. Il n'est donc pas possible d'effectuer des analyses sur site. D'autre part, cette technologie est cantonnée à l'étude de certains isotopes dont la nature du noyau se prête à l'analyse RMN. En particulier, l'analyse de noyaux atomiques à spin nucléaire nul n'est pas possible, ce qui est le cas dans certains minerais par exemple. D'autre part, parmi les matériaux organiques, certains rapports isotopiques ne sont pas directement accessibles, comme par exemple le rapport isotopique du carbone ¹²C/¹³C, ou les rapports isotopiques de l'oxygène ¹⁷O/¹⁶O et ¹⁸O/¹⁶O, car l'analyse RMN du carbone 12 ou de l'oxygène 16 ne sont pas possibles.

La présente invention présente un dispositif de détermination de l'origine d'un produit naturel ou élaboré, qui n'est pas limité aux produits organiques. La détermination, également basée sur l'estimation d'un ou plusieurs rapport(s) isotopique(s) du produit, permet de pallier les inconvénients précités. Elle met en oeuvre pour cela une analyse spectroscopique infrarouge du produit.

Plus précisément, l'invention présente un dispositif de détermination de l'origine d'un produit naturel ou élaboré par estimation d'au moins un rapport isotopique dudit produit, caractérisé en ce qu'il comprend :
- des moyens d'analyse spectrale dans l'infrarouge dudit produit permettant de déterminer les caractéristiques spectrales d'au moins une première liaison chimique dudit produit ou d'un de ses sous-produits, et celles d'au moins une liaison associée à la première liaison, c'est-à-dire comprenant un isotope d'au moins un des atomes formant ladite première liaison,
- des premiers moyens de comparaison desdites caractéristiques spectrales à une première base de données comprenant les caractéristiques spectrales de ladite première liaison chimique et de la liaison associée, afin d'établir au moins un rapport isotopique égal au rapport des quantités de sous-produit et de sous-produit associé composant ledit produit et comprenant respectivement ladite première liaison et ladite liaison associée,
- des seconds moyens de comparaison dudit rapport isotopique à une seconde base de données comprenant des valeurs dudit rapport isotopique pour des produits ou sous-produits d'origine connue, afin de déterminer l'origine dudit produit.

Le dispositif met en oeuvre des moyens accessibles, de type spectromètre infrarouge, qui permettent une utilisation sur site. D'autre part, il est possible par cette technique d'établir un très grand nombre de rapports isotopiques, ce qui permet d'étudier une grande diversité de produits.

D'autres avantages et caractéristiques de l'invention apparaîtront plus clairement à la lecture de la description qui suit, illustrée par les figures annexées qui représentent :
- les figures 1A à 1C, des spectres infrarouges illustrant les modifications dues à la présence de certains isotopes dans des liaisons chimiques de l'éthanol;
- la figure 2, un schéma représentant un exemple de dispositif selon l'invention ;
- les figures 3A à 3D, des courbes illustrant par un exemple simple une façon de calculer un rapport isotopique grâce au dispositif selon l'invention.

Grâce au dispositif selon l'invention, on cherche à déterminer l'origine d'un produit, soit naturel, c'est-à-dire prélevé directement dans la nature, soit élaboré, c'est-à-dire après mélange, transformation ou synthèse. Il peut s'agir par exemple de produits agro-alimentaires, tels que le vin, les alcools, le café, les huiles, etc. dont on cherche à connaître l'origine géographique, soit du produit lui-même, soit des sous-produits les constituant (par exemple, l'origine de l'alcool dans le vin afin d'en vérifier la chaptalisation). II peut s'agir aussi de produits non organiques, comme les minerais, dont on veut connaître l'origine géographique ou la date d'extraction. Il apparaît en effet que la concentration de certains isotopes varie en fonction des événements climatiques, physiques ou biochimiques. Par exemple, la concentration en deutérium, isotope de l'hydrogène dans l'eau, n'est pas partout la même, et ces variations se retrouvent dans les sols, les pluies et la végétation.

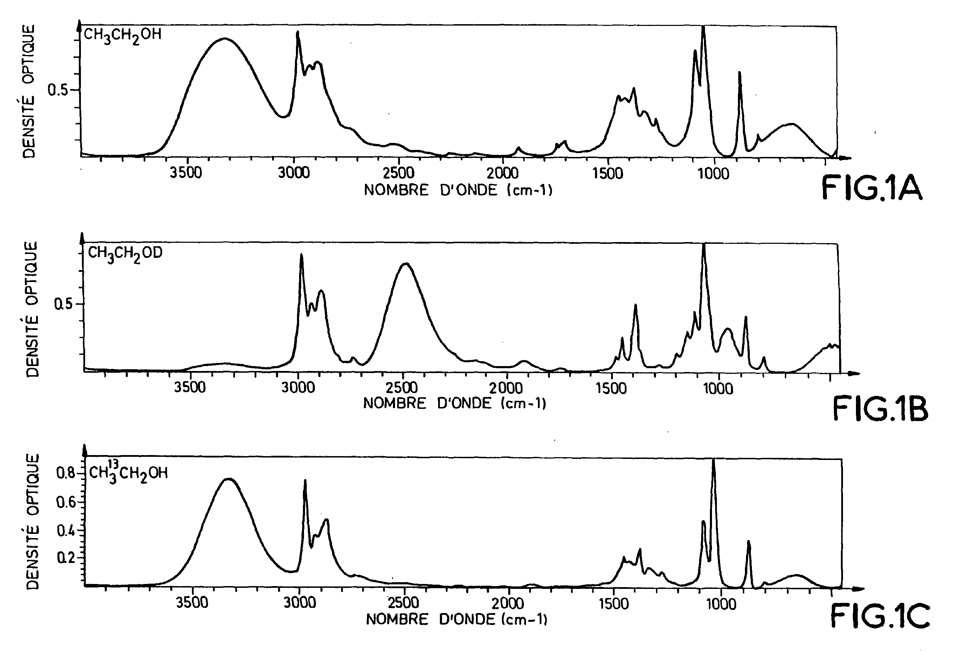

L'invention consiste en un dispositif permettant de déterminer l'origine d'un produit grâce à un appareillage simple, peu encombrant, et permettant d'accéder à une grande variété de produits. Cette détermination est réalisée, selon l'invention, au moyen de la spectroscopie infrarouge. Il apparaît en effet que la présence d'isotopes se traduit sur le spectre d'absorption infrarouge par le déplacement de bandes associées aux liaisons chimiques auxquelles ces isotopes participent. Ceci est illustré par un exemple particulier sur les figures 1A à 1 C.

Les figures 1A à 1C représentent respectivement des spectres infrarouges de l'éthanol (formule chimique CH₃CH₂OH), de l'éthanol sur lequel est substitué l'hydrogène en position OH par un deutérium (formule chimique CH₃CH₂OD) et de l'éthanol sur lequel est substitué le carbone supportant la liaison OH par son isotope, le carbone 13 (formule chimique CH₃¹³CH₂OH). Les spectres donnent la densité optique en fonction du nombre d'onde σ (inverse de la longueur d'onde) en cm⁻¹. Le spectre de la figure 1A donne pour l'éthanol la position et l'intensité des pics principaux. On le considère ici comme le spectre de référence. Le spectre de la figure 1 B montre l'influence sur les caractéristiques spectrales d'une substitution par le deutérium. L'influence est ici mise en évidence sur le site OH. Deux régions spectrales sont principalement concernées. Entre 3500 cm⁻¹ et 3000 cm⁻¹, le pic principale disparaît pratiquement. Entre 2600 cm⁻¹ et 2300 cm⁻¹ on observe l'apparition d'une nouvelle structure caractéristique de la substitution isotopique. Il s'agit d'un déplacement global depuis la région 3500 cm⁻¹ - 3000 cm⁻¹. De la même façon, des modifications des caractéristiques spectrales peuvent être mises en évidence lorsque la substitution par le deutérium se fait sur un atome d'hydrogène porté par une liaison CH. Le spectre de la figure 1C montre l'influence sur les caractéristiques spectrales d'une substitution par le carbone 13. Elle est mise en évidence sur le carbone supportant le groupement OH. L'allure générale du spectre reste inchangée par rapport au spectre de la référence de la figure 1A, cependant un déplacement caractéristique de l'ordre de 20 cm⁻¹ est visible sur le pic à 1040 cm⁻¹.

Cet exemple montre dans le cas particulier de l'alcool, que les modifications induites par la présence de deutérium dans la molécule sont très facilement détectables car elles portent sur des raies intenses et provoquent des déplacements de plusieurs centaines de cm⁻¹. Les modifications apportées par la présence de l'isotope ¹³C sont plus faibles mais sont détectables et très intéressantes car elles permettent grâce au dispositif selon l'invention, une mesure directe du rapport isotopique ¹³C/¹²C, mesure qui n'est par exemple pas directement possible d'effectuer par une analyse RMN.

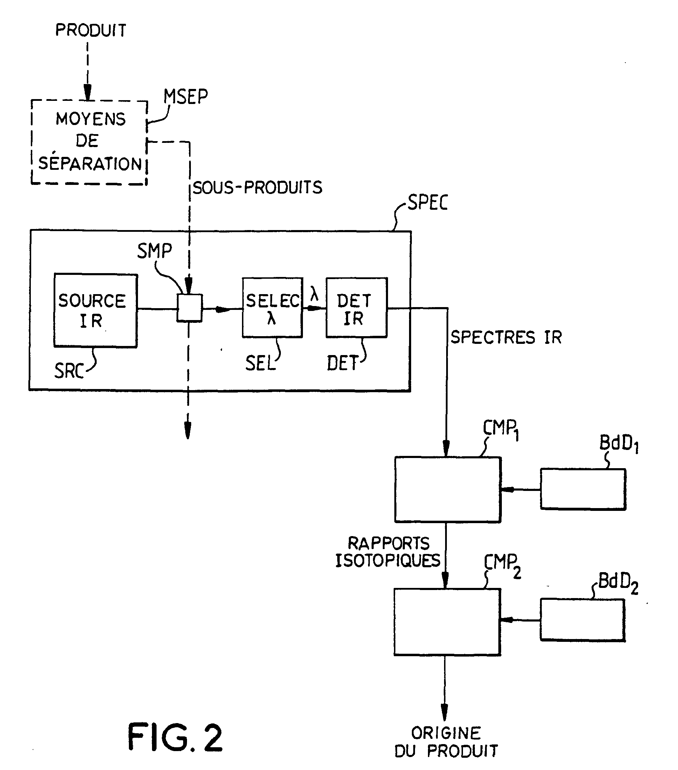

La figure 2 illustre par un schéma un exemple de dispositif selon l'invention. Le dispositif selon l'invention comprend des moyens d'analyse spectrale dans l'infrarouge SPEC du produit que l'on veut analyser, et des moyens de traitement assurés par exemple par un ordinateur.

Les moyens d'analyse spectrale SPEC sont dans cet exemple formés d'un spectromètre infrarouge classique. Il est équipé d'une ou plusieurs sources infrarouges couvrant le domaine spectral compris sensiblement entre 4000 et 400 cm⁻¹ (soit entre 2,5 et 25 µm), ce qui permet d'analyser la plupart des liaisons chimiques des molécules. Pour certaines applications nécessitant une plus grande sensibilité, ou dans le cas de l'analyse de produits gazeux qui présentent des raies d'absorption très fines, on peut également utiliser des diodes laser pour procéder à l'analyse spectrale. Le faisceau infrarouge est envoyé à travers le support d'échantillon SMP comprenant le produit à analyser, puis un dispositif de sélection en longueur d'onde SEL, de type monochromateur, permet d'envoyer vers des moyens de détection infrarouge DET, le faisceau transmis par l'échantillon à chacune des longueurs d'onde. Selon une variante, les moyens de détection sont formés d'une barrette de détecteurs élémentaires, ce qui permet d'établir en une seule mesure le spectre infrarouge total de l'échantillon. Selon l'invention, les moyens d'analyse spectrale donnent les caractéristiques spectrales de liaisons chimiques permettant d'établir par la suite un rapport isotopique. Il s'agit donc de déterminer les caractéristiques spectrales d'une première liaison chimique du produit ou d'un de ses sous-produits, et d'une liaison chimique associée. Par liaison chimique associée, on entend ici une liaison chimique comprenant un isotope d'au moins un des atomes formant la première liaison. En pratique, ces caractéristiques spectrales peuvent être obtenues par la prise directe d'un spectre infrarouge du produit que l'on veut analyser. Selon une variante, on procède au préalable à la séparation du produit en sous-produits grâce à des moyens de séparation MSEP (en pointillé sur la figure 2), et les caractéristiques spectrales des liaisons chimiques sont obtenues par la prise des spectres infrarouges des sous-produits. La séparation est par exemple obtenue par chromatographie ou par distillation. Ainsi, dans le cas par exemple de la détermination de l'origine d'un vin en vue de son authentification, on pourra établir directement un spectre infrarouge du vin, ou procéder à une séparation préalable en sous-produits (principalement eau et éthanol), et établir les spectres infrarouges des sous-produits.

Le dispositif selon l'invention présente l'avantage d'être adapté au produit sous toutes ses formes. En particulier, l'analyse d'un produit solide ne demande pas de mise en oeuvre particulière des moyens d'analyse spectrale.

L'exemple de la figure 2 présente en outre une variante intéressante dans le cas où les produits ou les sous-produits seraient des fluides. Selon cette variante en effet, les moyens d'analyse spectrale SPEC comprennent des moyens de circulation du fluide (symbolisés sur la figure 2 par une flèche en pointillé traversant le support d'échantillon SMP), permettant une analyse en continu, séquentielle, des sous-produits qui ont été séparés au préalable par les moyens de séparation MSEP. Cela permet ainsi une analyse des sous-produits en continu, qui, outre le fait qu'elle permet un gain de temps important dans l'analyse spectrale, peut par exemple s'appliquer à du contrôle de procédé.

Il est à noter que l'analyse spectroscopique ne demandant pas de façon systématique de préparation de l'échantillon, le dispositif selon l'invention peut être mis en oeuvre in situ, par exemple pour contrôler l'évolution d'un produit lorsqu'on ajoute un constituant. Dans cet exemple encore, les moyens de circulation peuvent être mis en oeuvre, dans le cas où le produit serait un fluide, pour procéder à une analyse en continu.

Les moyens de traitement comportent deux étapes. Des premiers moyens de comparaison CMP₁ à une première base de données BdD₁, permettant de déterminer le ou les rapports isotopiques dont on a besoin pour procéder à la détermination de l'origine du produit, et des seconds moyens de comparaison CMP₂ à une seconde base de données BdD₂, permettant la détermination de l'origine du produit.

La première base de données BdD₁ comprend, en fonction des rapports isotopiques recherchés, les caractéristiques spectrales de certaines liaisons chimiques et des liaisons chimiques associées, c'est à dire des liaisons similaires mais qui comprennent un isotope d'au moins un des atomes des premières liaisons. En pratique, la base de données BdD₁ comprend par exemple des spectres infrarouges de sous-produits du produit à analyser, qui comprennent lesdites liaisons chimiques, et des sous-produits associés qui comprennent les liaisons associées. Par exemple, dans le cas où le dispositif serait utilisé pour l'authentification de vins, la base de données BdD₁ peut comprendre le sous-produit éthanol de formule chimique CH₃CH₂OH, et des sous-produits associés de formules chimiques CH₃CH₂OD (isotope deutérium de l'hydrogène sur la liaison OH), CH₃¹³CH₂OH (isotope ¹³C du carbone sur le carbone porteur de la liaison OH), etc., chacun de ces sous-produits, comme nous l'avons vu précédemment, présentant un spectre infrarouge distinct. La comparaison du spectre infrarouge mesuré par les moyens d'analyse spectrale avec les spectres de la base de données BdD₁ permet alors de déterminer les rapports isotopiques, c'est-à-dire les rapports de quantités de sous-produits contenus dans le produit à analyser et comprenant respectivement une liaison chimique et la liaison chimique associée. Ainsi, dans l'exemple précédent, la comparaison du spectre infrarouge du vin à la base de données BdD₁ permet par exemple de déterminer un rapport isotopique D/H, égal au rapport de la quantité d'éthanol de formule CH₃CH₂OH et de la quantité d'éthanol de formule CH₃CH₂OD contenues dans le vin à analyser. Elle permet également selon cet exemple de déterminer un rapport isotopique ¹³C/¹²C, égal au rapport de la quantité d'éthanol de formule CH₃CH₂OH et de la quantité d'éthanol de formule CH₃¹³CH₂OH contenues dans le vin à analyser. La base de données BdD1 sera d'autant plus importante que le nombre de rapports isotopiques dont on a besoin pour déterminer l'origine du produit est grand.

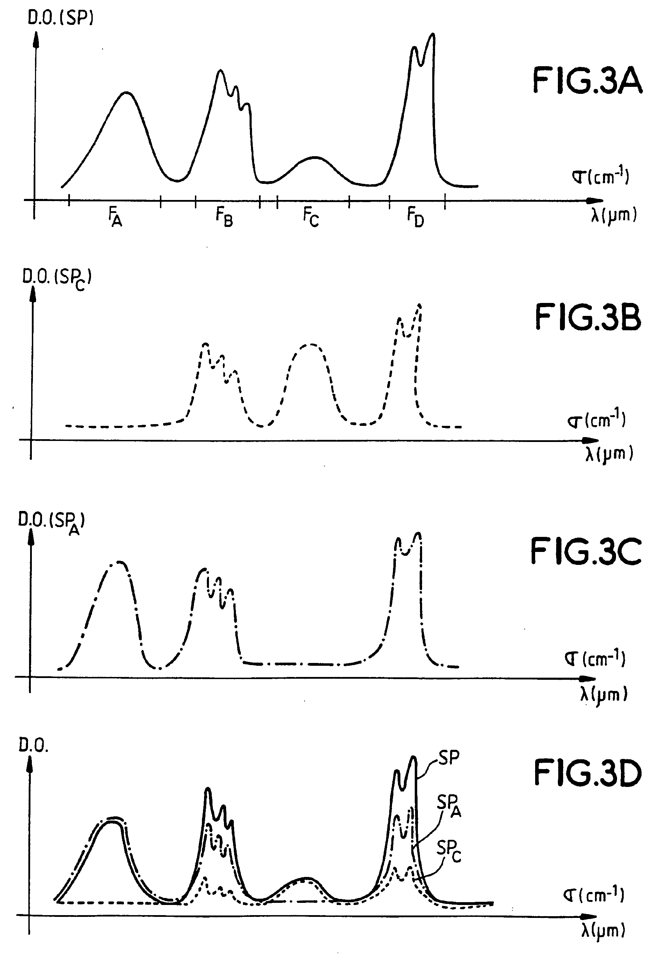

Les figures 3A à 3D montrent selon un exemple très simplifié comment on peut, à partir des spectres infrarouges de la base de données BdD1, déterminer un rapport isotopique du produit dont on cherche l'origine. La figure 3A représente par exemple le spectre infrarouge (densité optique DO en fonction du nombre d'onde σ) d'un sous-produit SP contenu dans le produit à étudier, mesuré avec les moyens d'analyse spectrale. Le spectre fait apparaître quatre liaisons chimiques A, B, C, D, la liaison C étant une liaison associée à la liaison A. Les caractéristiques spectrales de chacune de ces liaisons apparaissent dans des fenêtres spectrales notées respectivement F_{A}, F_{B}, F_{C}, F_{D}. Le sous-produit SP est un mélange d'un premier sous-produit SP_{A}, contenant la liaison A, et d'un sous-produit SP_{C}, contenant la liaison C et donc associé au sous-produit SP_{A}. On recherche dans cet exemple le rapport isotopique noté A/C, égal au rapport des quantités respectives de sous-produit SP_{A} et SP_{C} dans le sous produit SP. Pour cela, on enregistre dans la base de données BdD₁ les spectres infrarouges des sous-produits SP_{A} (figure 3C) et SP_{C} (figure 3B) mesurés dans les mêmes conditions que le spectre infrarouge du sous-produit SP illustré sur la figure 3A. On recherche alors quelle combinaison linéaire des spectres des sous-produits contenus dans la base de données permet d'ajuster au mieux le spectre du sous-produit SP. La figure 3D montre ainsi 4 courbes référencées respectivement SP (en trait plein), SP_{A} (en trait pointillé alterné court et long) et SP_{C} (en trait pointillé court). La courbe SP est le spectre infrarouge du sous-produit SP (identique à celui de la figure 2A), la courbe SP_{A} est le spectre infrarouge du sous-produit SP_{A} affecté d'un premier coefficient et le spectre SP_{C} est le spectre du sous-produit SP_{C} affecté d'un second coefficient, les coefficients étant déterminés pour que la somme des courbes SP_{A} et SP_{C} donne sensiblement la courbe SP. Le rapport isotopique A/C recherché est alors donné par le rapport des coefficients. Bien entendu, cet exemple est volontairement très simplifié pour faire mieux comprendre le principe du calcul. En pratique, on sera amené à rechercher plusieurs rapports isotopiques, mettant en oeuvre plusieurs liaisons chimiques associées faisant apparaître des modifications spectrales multiples et parfois assez peu faciles à discerner. On peut alors utiliser des méthodes d'optimisation classiques pour déterminer les coefficients qui permettront d'établir les rapports isotopiques, basées par exemple sur une méthode itérative suivie d'une minimisation de l'erreur entre la courbe mesurée et la combinaison de courbes testée.

Dans des cas complexes, il est également avantageux de limiter le traitement à des fenêtres spectrales déterminées en fonction des rapports isotopiques que l'on recherche, plutôt que de travailler sur les spectres dans leur intégralité. Ainsi, dans l'exemple des figures 3A à 3D, on peut limiter la comparaison du spectre SP avec les spectres SP_{A} et SP_{C} aux fenêtres notées F_{A} et F_{C} et correspondant aux liaisons chimiques A et C associées.

Les seconds moyens de comparaison CMP₂ à une seconde base de données BdD₂ du rapport isotopique déterminé lors de la première étape de comparaison précédemment décrite, permettent finalement de déterminer l'origine du produit. Pour cela, la seconde base de données comprend des valeurs du rapport isotopique pour des produits ou sous-produits d'origine connue. Ainsi, dans le cas par exemple de l'application du dispositif à l'authentification des vins, on pourra faire une comparaison des rapports isotopiques deutérium/hydrogène sur les différents sites de l'alcool éthylique. Le rapport deutérium/hydrogène entre les sites 1 et 2 (rapport R) de la molécule (respectivement site CH₃ et site CH₂) s'est également avéré significatif. Ainsi, l'étude comparative de nombreux échantillons a pu montrer, par exemple, que la mesure du rapport deutérium/hydrogène sur le site 1 caractérise principalement l'espèce végétale qui a synthétisé le sucre, et, dans une moindre mesure, la nature de l'eau utilisée par la plante au cours de la photosynthèse. La mesure du rapport deutérium/hydrogène sur le site 2 donne des indications sur les conditions météorologiques du lieu de production du raisin et sur la concentration en sucre du moût initial.

Bien entendu, la nature des informations contenues dans la seconde base de données BdD₂ dépend de la nature du produit dont on cherche à déterminer l'origine. La fiabilité du dispositif augmente avec le nombre de données mises en mémoire dans ladite base.

## Revendications

1. Dispositif de détermination de l'origine d'un produit naturel ou élaboré par estimation d'au moins un rapport isotopique dudit produit, caractérisé en ce qu'il comprend :
- des moyens d'analyse spectrale (SPEC) dans l'infrarouge dudit produit permettant de déterminer les caractéristiques spectrales d'au moins une première liaison chimique dudit produit ou d'un de ses sous-produits, et celles d'au moins une liaison associée à la première liaison, c'est-à-dire comprenant un isotope d'au moins un des atomes formant ladite première liaison,
- des premiers moyens de comparaison (CMP₁) desdites caractéristiques spectrales à une première base de données (BdD₁) comprenant les caractéristiques spectrales de ladite première liaison chimique et de la liaison associée, afin d'établir au moins un rapport isotopique égal au rapport des quantités de sous-produit et de sous-produit associé composant ledit produit et comprenant respectivement ladite première liaison et ladite liaison associée,
- des seconds moyens de comparaison (CMP₂) dudit rapport isotopique à une seconde base de données (BdD₂) comprenant des valeurs dudit rapport isotopique pour des produits ou sous-produits d'origine connue, afin de déterminer l'origine dudit produit.

2. Dispositif selon la revendication 1, caractérisé en ce que les caractéristiques spectrales sont déterminées par la mesure d'un spectre infrarouge global dudit produit.

3. Dispositif selon la revendication 1, caractérisé en ce qu'il comprend en outre des moyens de séparation préalable (MSEP) du produit en sous-produits et en ce que les caractéristiques spectrales sont déterminées par la mesure de spectres infrarouges desdits sous-produits.

4. Dispositif selon l'une des revendications 2 ou 3, caractérisé en ce que ladite première base de données (BdD₁) comprend des spectres infrarouges de sous-produits dudit produit comprenant au moins une desdites premières liaisons et de sous-produits associés auxdits sous-produits comprenant chacun une liaison associée à ladite première liaison, les spectres étant préalablement établis dans les mêmes conditions que celles de la mesure de spectres infrarouges du produit ou sous-produits lors de l'analyse spectrale, et en ce que chaque rapport isotopique est établi en déterminant la combinaison linéaire des spectres infrarouges du sous-produit et du sous-produit associé de ladite base de données permettant d'établir ledit rapport isotopique, qui ajuste le mieux le spectre infrarouge du produit ou du sous-produit mesuré.

5. Dispositif selon l'une des revendications précédentes, caractérisé en ce que les premiers moyens de comparaison (CMP₁) à la première base de données (BdD₁) sont limités à un nombre donné de fenêtres spectrales prédéterminées en fonction de la nature du ou des rapport(s) isotopique(s) que l'on cherche à établir.

6. Dispositif selon l'une des revendications précédentes, caractérisé en ce que les moyens d'analyse spectrale sont formés d'un spectromètre infrarouge comportant des moyens d'émission (SRC) dans la bande spectrale 4000 cm⁻¹ - 400 cm⁻¹.

7. Dispositif selon l'une des revendications précédentes, caractérisé en ce que le produit ou les sous-produits à analyser étant des fluides, les moyens d'analyse spectrale comprennent des moyens de circulation du fluide, permettant une analyse en continu, séquentielle, dudit produit ou desdits sous-produits.
